# EUROPEAN PATENT APPLICATION

(11) **EP 3 521 820 A1**
(43) Date of publication of application: **07.08.2019**
(21) Application number: 18155392.6
(22) Date of filing: 06.02.2018
(51) Int. Cl.: G01N 33/00, G01N 1/22, G01N 33/22

(54) **GAS ANALYSER**

(71) Applicant: ExTeVent AB, 47537 Bohus-Björkö (SE)
(72) Inventor: LEICHT, Mikael, 47537 Bohus-Björkö (SE)
(74) Representative: Ström & Gulliksson AB

(57) **Abstract**

A gas analyser (1, 2) has one or more body elements (10, 10'), each of which includes a sample bore (7) extending from a respective proximal surface (11) to a distal surface (12) of the body element (10, 10'); as well as gas conditioning means (25) configured to connect with the sample bore (7). At least one sample bore (7) is provided with an orifice (21) disposed between the proximal surface (11) and the distal surface (12), and gas analysing means (28) is arranged in communication with the orifice (21).

## Description

### TECHNICAL FIELD

The present invention relates to gas analysing assemblies and related methods.

### BACKGROUND

Analysis and detection of various gaseous components in this field is commonly associated with a number of challenges. Raw gas, such as natural gas or biogas, typically contains moisture which may cause corrosion and harm to analysing devices if condensed inside a gas analysing equipment. This is prone to occur if the gas analyser is placed in an environment where the temperature is lower than a corresponding dew-point temperature of the gas to be analysed.

The prior art mitigation to the problem of condensation is typically condensing the moisture from the gas to be analysed prior to supplying the gas to the gas analysing equipment. Prior-art systems include condensation of liquid from the gas by means of a condenser and a pump powered by a motor. However, an electric motor may potentially pose a hazard if installed e.g. in a gas handling facility in that leaked flammable gas surrounding the gas analysing equipment may be ignited.

Prior-art gas analysing equipment further exhibits shortcomings in terms of flexibility vis-a-vis the present task, in that these systems tend not to be purpose built, but rather offer generic multi-purpose type systems. A customer may thus pay dearly for functionality which is not required for the application at hand.

A further disadvantage associated with some prior-art systems which are voluminous becomes apparent in applications which place demands on the economy of space.

A yet further disadvantage of known systems is lack of such robustness which is required in applications on site, such as gas storage facilities, gas-plants and gas transmission installations.

In this regard, certain systems and methods are known for instance from US3976450A.

However, these known systems suffer from basically the same disadvantages as discussed above.

As such, there is a need for an improved gas analyser.

### SUMMARY

It is thus an object of the present invention to provide a gas analyser design which is improved over prior art and which can be purpose built with facilitated customization.

It is a further object of the present invention to provide a gas analyser design which enables analysis of raw gas e.g. gas which is saturated in terms of moisture content, with reduced risk of condensation.

It is a yet further object of the present invention to provide a gas analyser design in which sensors of the gas analyser are mechanically protected.

It is a still further object of the present invention to provide a cost-efficient gas analyser.

It is another object of the present invention to provide a mechanically robust gas analyser.

These objects, and further object which will appear from the following description, have now been achieved by the technique set forth in the appended independent claims; preferred embodiments being defined in the related dependent claims.

In a first aspect, there is provided a gas analyser which comprises one or more body elements, each of which including a sample bore extending from a respective proximal surface to a distal surface of the body element. Furthermore, each body element includes gas conditioning means configured to connect with the sample bore. At least one sample bore is provided with an orifice disposed between the proximal surface and the distal surface, and gas analysing means is arranged in communication with the orifice.

It is thus achieved that conditioning of the gas to be analysed and analysis of the gas are carried out in a single body element. It is further achieved that a gas to be analysed is continuously conditioned throughout the duration of the gas inside the gas analyser; including from the time the gas enters the gas analyser, during analysis of the gas and after analysis of the gas. It is further achieved that gas is being conditioned also at the point where it is actually analysed, further reducing the risk of condensation.

In an embodiment, the gas conditioning means are configured to connect with the sample bore upstream of the same, thereby assuring that gas to be analysed is duly conditioned prior interaction with the gas analysing means.

In a further embodiment, the orifice may open to a mounting bore configured to receive and/or hold the gas analysing means, thereby protecting gas analysing means and sensors thereof from mechanical harm. In addition, the gas analysing means, in particular the body element may provide a temperature conditioned environment for the sensors thereof, further improving accuracy of analysis and reducing the risk of condensation in the gas analysing means. Gas analysing means and equipment are thus connectable directly to the body element, rendering further mounting arrangements redundant.

A gas permeable disk may be disposed in the mounting bore. The gas to be analysed diffuses from the orifice to a detector of the gas analysing means.

In a further embodiment, each body element constitutes one entity. This drastically reduces the number of parts of the gas analyser and significantly improves the robustness of the same. Moreover, the cost-efficiency of the gas analyser is improved.

The body element may be manufactured from a solid casted body, which provides for uniform heat conductivity in the gas analyser. In addition, the gas analyser may be manufactured from a material having a high heat capacity, hence a solid body element will be less susceptible to temperature fluctuations and provide stable temperatures to a gas inside the body element.

In one embodiment, the means for gas conditioning is configured to condition a gas to be analysed to attain and/or maintain a temperature above a corresponding dew point temperature of the gas to be analysed. Thereby the gas analyser can analyse actual raw gas having a moisture content without risk of condensation and doing so in the absence of additional condensers, motors, pump etc.

The means for gas conditioning comprise a plurality of conditioning bores, the conditioning bores being configured to be fluidly interconnected. The gas conditioning bores may be independently formed in the body element. Thereby the gas analyser achieves integrated conditioning of a gas to be analysed, rendering separate temperature conditioning means and condensation preventing measures redundant.

In one embodiment, the conditioning bores are configured to be fluidly interconnected in serial fluid communication to form a continuous conditioning channel. Thereby the length of the conditioning channel is variable and thereby conditioning bores of several body elements may be aggregated to form a single conditioning channel.

Each conditioning bore may extend from the proximal surface to the distal surface of the body element. The proximal surface and the distal surface may be parallel opposing surfaces. Hence, this configuration facilitates a modular gas temperature conditioning means.

In one embodiment, respective inner walls of the sample bore and the means for gas temperature conditioning are heated by heating means adapted to heat the body element, typically during operation. The heating has the effect that the gas analyser (including the sampling bore) is maintained at a required temperature, thereby preventing condensation therein. The heating has the further effect that temperature of the gas to be analysed is held stable between different gas analysing means. The heating has the further effect that the gas analyser according to the disclosure is not required to be installed in a heated environment or compartment.

The conditioning bores and/or the sample bore may extend side by side and in parallel in the body element. This configuration facilitates that when body elements are connected in mating sequence, respective conditioning bores and sample bores of adjacent body elements fluidly connect in series.

In one embodiment the gas analysing means comprises one or more of a carbon dioxide detector, an oxygen detector, a methane detector, a hydrogen sulfide detector, thereby the gas analyser may analyse the composition of a gas to be analysed in respect of several components of the gas.

The gas analyser according the disclosure is not limited to a specific number of conditioning bores in each body element, however according to one embodiment the number of conditioning bores in each respective body element is between four to twelve, preferably six to ten, and most preferred eight, whereby a sufficient aggregated length of the conditioning channel is achieved to secure proper conditioning of the gas to an appropriate temperature. For example, by interconnecting eight conditioning bores, the gas G to be analysed may be sent back and forth eight times through the body element.

In a second aspect, there is provided a modular gas analysing assembly which comprises a gas analyser according to any one of the embodiments of the first aspect, wherein a desired/required functionality of the modular gas analysing assembly is attained by arranging the one or more body elements in mating sequence to connect respective sample bores and to connect respective conditioning bores. Thereby achieving flexibility in the choice of configuration of the gas analyser.

In a third aspect, there is provided a method for analysing gas, the method comprising: providing a plurality of independent conditioning bores; interconnecting said conditioning bores to form one or more conditioning channels; arranging gas analysing means in communication with the conditioning channel; heating the body element by means of a heating element; feeding a gas to be analysed to the conditioning channel; and analysing the gas to be analysed by means of the gas analysing means.

### BRIEF DESCRIPTION OF THE DRAWINGS

Embodiments of the present invention will now be described with reference to the accompanying drawings which illustrate non-limiting examples on how the embodiments can be reduced into practice and in which:
Fig. 1a is a diagrammatic side view representation of a gas analyser body element according to an exemplary embodiment of the disclosure,
Fig. 1b is a diagrammatic further representation of the body element of Fig.1a seen in the direction of the arrows 1 b-1 b,
Fig. 2a is a diagrammatic side view representation of a gas analyser body element according to a further exemplary embodiment of the disclosure,
Fig. 2b is a diagrammatic representation of the gas analyser body element of Fig. 2a seen in the direction of the arrows 2b-2b.
Fig.3 is a top view of the gas analyser body element shown in Fig. 2.
Fig. 4 is an isometric view of a gas analyser according to an exemplary embodiment of the disclosure.
Fig. 5 is an isometric view of a modular gas analysing assembly according to an exemplary embodiment of the disclosure.

### DETAILED DESCRIPTION

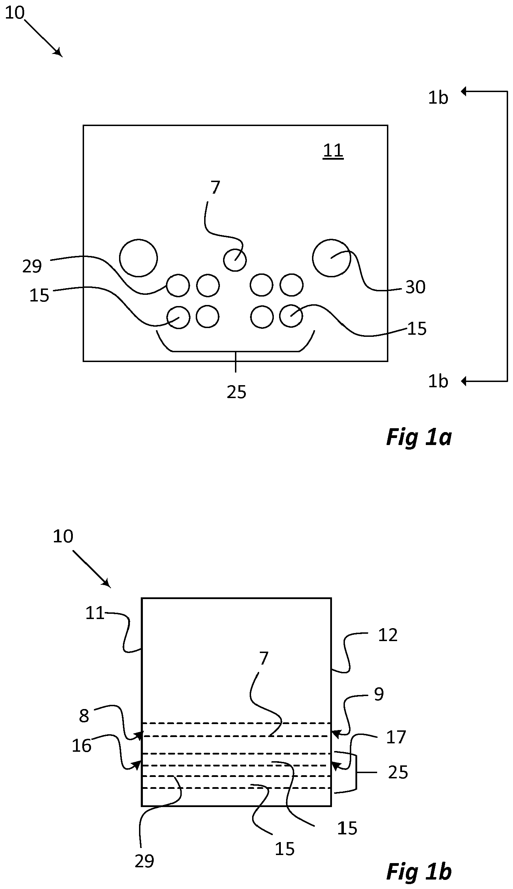

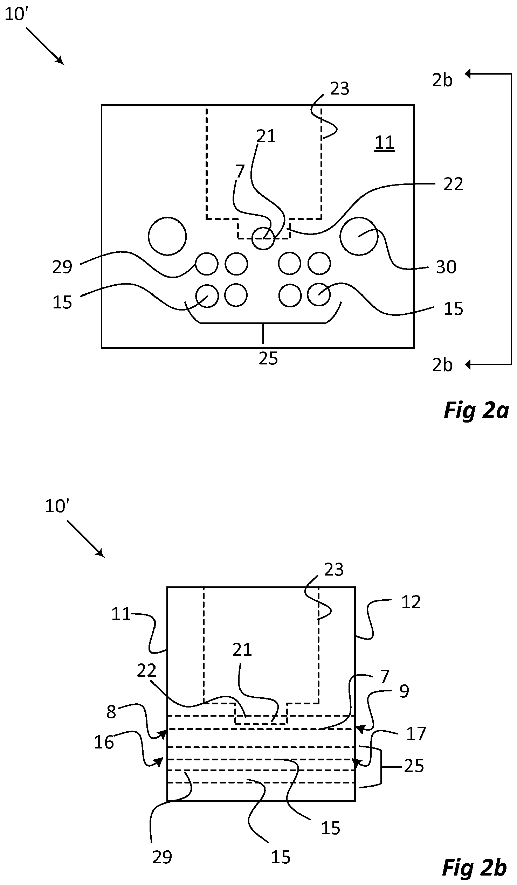

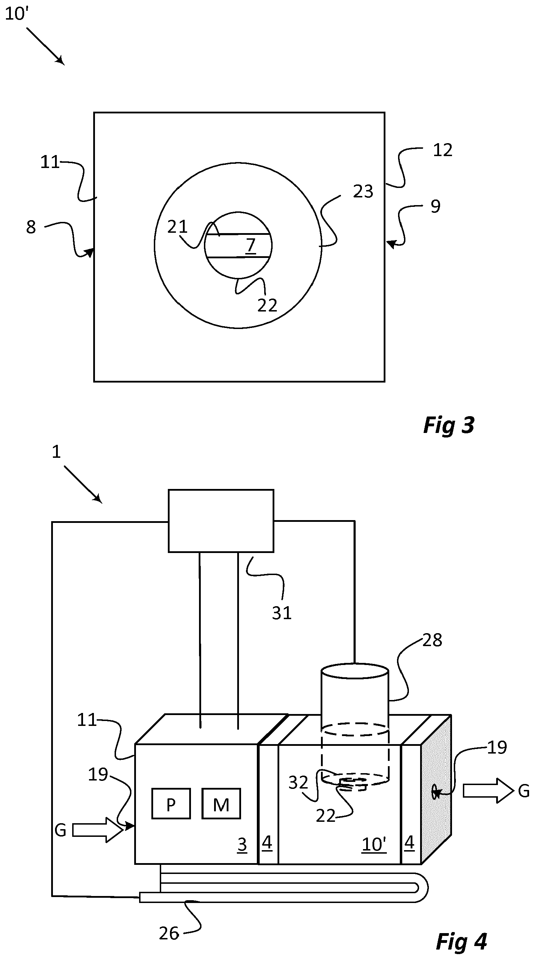

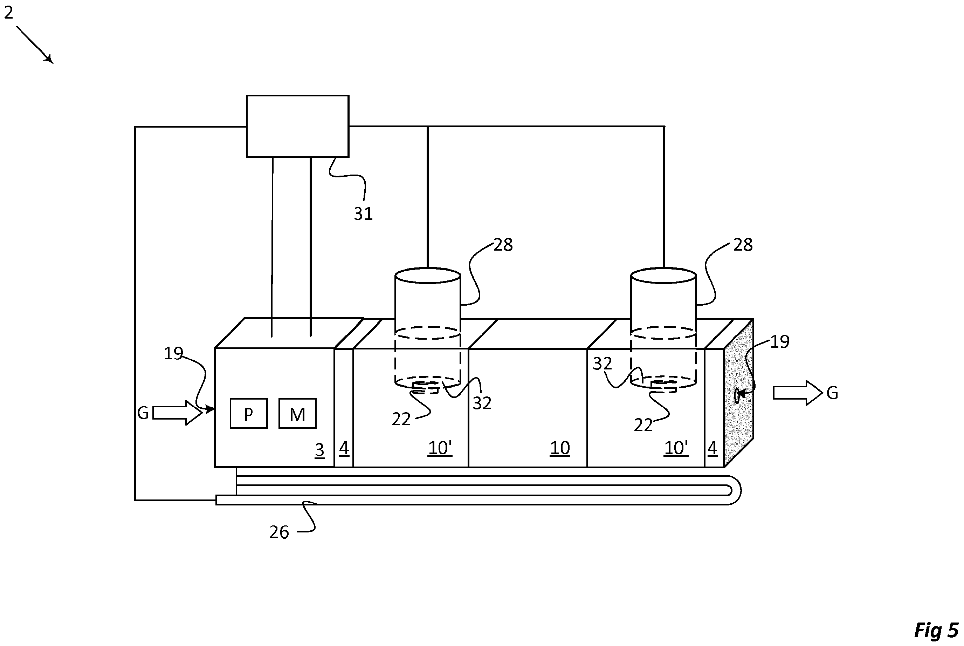

Referring to Figs 4 and 5, there is shown possible implementations of the gas analyser 1, 2 according to the present disclosure. These examples will be described further below. The features of the body elements 10, 10' are shown more in detail in Figs 1 and 2, where there is shown a gas analyser body element 10, 10' according to an exemplary embodiment of the disclosure. The body element 10, 10' constitutes a single entity. The structure of the body element 10, 10' may be attained by manufacturing, such as milling and/or drilling of a casted body. However, other methods of manufacture have been contemplated such as 3D-printing or casting.

A suitable material of the body element 10, 10' has been found to be acid resistant stainless steel to resist corrosion due to the gas to be analysed, however other suitable materials are contemplatable.

The body element 10, 10' comprises two parallel surfaces; a proximal surface 11 and a distal surface 12 which are parallel. An independently formed sample bore 7 extends continuously in one direction from an inlet opening 8 disposed at the proximal surface 11 to an outlet opening 9 disposed at the distal surface 12.

The body element 10, 10' comprises gas conditioning means 25 having a number of conditioning bores 15 configured to fluidly connect to the sample bore 7, upstream of the same. Typically, the gas conditioning means 25 connect to the inlet opening 8 of the sample bore 7, as will be further described herein.

The body element 10, 10' comprises gas conditioning means 25 in the form of a conditioning channel 19 (see Figs 4 and 5). The conditioning channel 19 is comprised of a plurality of interconnected conditioning bores 15.

The conditioning bores 15 extend from a proximal surface 11 to a distal surface 12. Each conditioning bore 15 extends continuously in one direction from a respective inlet opening 16 disposed at the proximal surface 11 to a respective outlet opening 17 disposed at the distal surface 12.

The configuration of the proximal surface 11 and the distal surface 12 thus allows for one or more body elements 10, 10' to be arranged in mating sequence, hence by arranging a respective distal surface 12 of a body element 10, 10' to mate with a respective proximal surface 11 of a further body element 10, 10' (see Figs 4 and 5).

Furthermore, the configuration of the proximal surface 11 and the distal surface 12 in parallel thus allows for respective sample bores 7 of more than one body element 10, 10' to be connected in serial fluid communication.

The configuration of the proximal surface 11 and the distal surface 12 thus allows for respective conditioning bores 15 of more than one body element 10, 10' to be connected in serial fluid communication.

The body elements 10, 10' may be uniform in terms of shape, facilitating flexibility and ease of assembly of the gas analyser 1, 2. In particular, the body elements 10, 10' may be substantially cubical.

By arranging one or more body elements 10, 10' in mating sequence, as depicted in Fig. 5, hence by arranging a respective distal surface 12 of a body element 10, 10' to mate with a respective proximal surface 11 of a further body element 10, 10', respective sample bores 7 are connected to form aggregated sample bores 7 extending through more than one body element 10, 10'. Correspondingly, conditioning bores 15 extending side by side in parallel, are connected to form aggregated conditioning bores 15 extending through more than one body element 10, 10'. By further interconnecting the aggregated conditioning bores 15, the plurality of conditioning bores 15 are interconnected to form a continuous conditioning channel 19 (see Figs 4 and 5).

In the described example, this design comprises the configuration of end pieces 4 disposed respectively at a proximal surface 11 and a distal surface 12, in particular mating with a proximal surface 11 and distal surface 12 respectively. The end pieces 4 illustrated in Figs 4 and 5 are configured to receive fluid, i.e. gas G from a conditioning bore 15 and diverting said gas G received into a further conditioning bore 15, in particular into an inlet opening 16 of a conditioning bore 15 or an outlet bore 17 of a conditioning bore 15, thus diverting the direction of the fluid flow 180 degrees. Thereby, the gas flow G is sent back and forth through the conditioning bores 15 of the body elements 10, 10' between end pieces 4 in order to achieve the appropriate temperature. Finally, one of the end pieces 4 receives the conditioned gas G from the gas conditioning means 25 and feeds it into the sample bore 7. However, the sample bore 7 also contributes to the conditioning of the gas G, as will be apparent herein.

Accordingly, a gas G to be analysed is provided to the gas conditioning means 25, e.g. by feeding the gas G to one of the conditioning bores 15, in particular the first conditioning bore 15 of the plurality of interconnected conditioning bores 15. The direction of a gas flow G is thus defined as from a first conditioning bore 15 to a last conditioning bore 15 of the interconnected conditioning bores 15.

The number of conditioning bores 15 formed in each body element 10 may be determined for example based on a number of parameters, including but not limited to: a corresponding dew point of a gas G to be analysed, the number of body elements 10 arranged in mating sequence i.e. an aggregated length of the conditioning bores 15, a radius of the conditioning bores 15, a material of the body element 10, heat conductive properties of the body element 10, 10' and power of a heating means 26.

As shown in Figs 4 and 5, heating means 26 are provided to heat the body elements 10, 10' of the gas analyser 1, 2. It follows that by providing heat to the body elements 10, 10' by the heating means 26, that the heat conductive material of the body elements 10, 10' efficiently conduct the heat to the conditioning bores 15 and the sample bore 7 which are thus heated. It also follows that heat is transferred directly from internal walls of the conditioning bores 15 and the sample bore 7 to the gas G to be analysed. It is thereby accomplished that the gas G to be analysed is conditioned to attain and/or maintain a required temperature sufficient to prevent condensation of liquid from the gas G to be analysed. It is further accomplished that conditioning of the gas G is carried out by each body element 10, 10' of the gas analyser 1, 2. Another accomplishment is that the substantial thickness of the body elements 10, 10' facilitates stable provision of heat to the gas conditioning means 25.

The heating means 26 are preferably disposed in or in close proximity of the body elements(-s) 3, 4, 10, 10' of the gas analyser 1, 2. In a preferred embodiment, the heating means 26 comprise an electrical heating arrangement, such as heat tracing disposed at one or more surfaces of the gas analyser 1, 2. The heating means 26 may be of the type generating heat by providing internal resistance. In the embodiment described herein, the heating means 26 comprises heat tracing disposed directly underneath the gas analyser 1, 2.

It is thus accomplished that the gas analyser 1, 2 analyses gas having a moisture content in the absence of / independent any type of means which removes or significantly reduces the moisture content of the gas G to be analysed. It is further accomplished that the gas analyser according to the present disclosure can operate in such environments which are associated with or classified as hazardous with regards to risk of explosion. Such environments may include sites for production, distribution or storage of combustible gas not limited to natural gas, sludge digestion gas, liquefied natural gas, liquefied compressed natural gas.

The gas analyser 1, 2 of Figs.4-5 may for example be provided directly on a gas transmission pipe (not shown).

Raw gas, such as gas produced by a biogas plant is typically has a temperature about 35°C and is saturated at a moisture content corresponding to 4-5% of the volume. It follows that if the temperature of the gas is lowered at some point e.g. in gas analysing equipment, the gas will not be able to hold the same amount of moisture and thus, condensation will occur. Therefore, the gas analyser 1, 2 conditions, by means of the gas conditioning means 25 and maintains, by means of the sample bore 7, the gas at an elevated temperature being above the temperature of dew point for the applicable moisture content of the gas G to be analysed. The gas analyser 1, 2 thereby further achieves that the same gas G composition for which flow is measured by means of flow meter M is also the gas G composition which is subsequently analysed by gas measuring means 28.

According to a further aspect, the disclosure relates to a method for analysing gas. The method is in particular suitable for analysing gas having a moisture content, i.e. raw gas which is typically damped gas as described above.

Referring again to Figs 2a-b, there is shown an exemplary body element 10' having the features corresponding to the exemplary body element 10 as explained with reference to Figs 1a and 1b. The body element 10' of Figs 2a-2b is provided with additional means for extracting gas to be analysed from the sample bore 7. The gas analyser 1, 2 comprises one or more such body elements 10'. In the body element 10' of Figs 2a-b the sample bore 7 is provided with an orifice 21 for extracting conditioned gas from the sample bore 7. The orifice 21 is disposed between, the proximal surface 11 and the distal surface 12, typically midway between.

In this exemplary embodiment, the body element 10' further comprises a test cell 22 which is illustrated in Figs. 2a, 2b and 3, in the form of a bore having a center axis which is transversal a center axis of the sample bore 7. However, other angles are conceivable. The test cell 22 extends substantially halfway through the sample bore 22, and the sample bore 7 thereby merges with the sample bore 7 such that the transversal arrangement gives the orifice 21 the shape of a half cylinder, i.e. a cylinder divided along its longitudinal length. The orifice 22 thereby has a cross-sectional area calculated as the diameter of sample bore times the diameter of the test cell 22. From the test cell 22, gas G to be analysed diffuses 22 into the gas analysing means 28, as will be explained further herein.

As previously mentioned, the sample bore 7 serves as a conditioning bore similar to the conditioning bores 15. Since the sample bore 7 and the orifice 21 are integrally formed in the body element 10, 10', also the sample bore 7 effectively contributes to the conditioning.

As shown in Fig.5, at least one body element 10' is provided with gas analysing means 28, and the sampling bore 7 is provided with an orifice 21 in communication with the gas analysing means 28.

The orifice 21 provides fluid communication between the sample bore 7 and a mounting more 23 (see Figs 2a-b). The mounting bore 23 may be a drilled bore, and it is configured to slidably receive a gas analysing means 28, such as a gas detector (not shown). Preferably, the mounting bore 23 is formed coaxially with the test cell 22. By arranging the gas analysing means 28 in the mounting bore 23, the interior of the gas analysing means 28 is heated through heat transfer from the body element 10, 10'.

The gas analysing means 28 is received in the mounting bore 23 in sealing engagement, for example by means of sealing means such as an O-ring (not shown). Thereby, gas G is not expelled through the mounting bore 23. A gas permeable disk 32 is disposed in the mounting bore 23 to allow for gas G to diffuse from the orifice 21 to the mounting bore 23 where the gas analysing means 28 are arranged. In particular a sintered disk is arranged between the test cell 22 and an interior of the gas analysing means 28 where the gas detector of the gas analysing means 28 is disposed. Gas G subsequently diffuses from the test cell 22 to the detector through the sintered disk, thereby protecting the gas detector.

It is thus accomplished that the gas analysing means 28, i.e. detectors of gas analysing means 28 are protected. It is further accomplished that analysis of a gas is carried out without disrupting the gas flow G through the sample bore 7; gas to be analysed is extracted from the sample bore 7 by the orifice 21 without deflecting a main flow of gas G through the sample bore 7. The gas analyser 1, 2 thus performs in-flow / non-disruptive extraction of a sample of gas G to be analysed.

The mounting bore 23 may additionally be configured to hold the gas analysing means 28 for example in a vertical or horizontal position of the gas analysing means 28.

As shown in Figs 1-2, the body element 10, 10' comprises assembly bores 30 disposed on each side of the conditioning bores 15 and extending from the proximal surface 11 to the distal surface 12. The assembly bores 30 are configured to receive assembling means such as rods or threaded elements (not shown). The assembly bores 30 facilitate that a plurality of body elements 10, 10' arranged in mating sequence are held together by inserting said assembling means extending through all of the respective assembly bores 30 of the plurality of body elements 10, 10' and subsequently providing bolts or the like tensioning means to the respective ends of the rods such as to exert pressure on the gas analyser 1, 2 and the sequence of body elements 10, 10'.

According to aspects, one or more or all of the conditioning bores 15, sample bore 7, test cell, mounting bore, assembly bores 30 are drilled circular bores.

With reference to Fig.5, the gas analysing means 28 may as explained comprise one or more gas analysing detectors associated with a respective body element 10'. The gas analyser 1, 2 of the present disclosure is particularly suitable for use with methane detector, carbon dioxide detector, oxygen detector and hydrogen sulfide detector.

The gas analyser 1, 2 is typically provided with further conditioning means. Further conditioning means may include but not limited to pressure regulating means P and flow regulating and/or meter means M (see Figs 4-5). The further conditioning means are preferably configured upstream the gas conditioning means 25, and the further conditioning means may be connected to one or more further body elements 3 which are compatible with body element 10, 10' and thus can be arranged in mating sequence with body elements 10, 10'. It follows that raw gas to be analysed and which enters the gas analyser 1, 2 may be conditioned in respect of pressure by means of a pressure regulating device P and measured and/or regulated in respect of flow by means of a flow meter device M prior it is conditioned with respect of temperature by the gas conditioning means 25, where the gas conditioning means 25 are gas temperature conditioning means. The pressure regulating device P and/or the flow meter device M may be disposed in one or more further conditioning body elements 3 which are compatible, i.e. in mating sequence, with the body elements 10, 10' of the gas analyser 1 and end pieces 4. The flow meter device M may also regulate the flow of gas G.

The gas analyser 1, 2 according to the disclosure further accomplishes that the gas analyser 1, 2 includes correction against the effects of changes in pressure and temperature.

A control system 31 is connected to the gas analyser 1, 2 for monitoring and controlling the gas analyser 1, 2, this is shown in Figs 4-5.

Reference is now made to Fig. 5 which shows an exemplary modular gas analyser 2. As have been previously explained in relation to Figs 1a-2b, the gas analyser 1 may comprise more than one body element 10'. Typically, the body element 10 depicted in Figs1a-b constitutes a spacing body element interposed between two adjacent body elements of the type depicted in Figs 2a-b comprising an orifice 21 for gas G extraction. In this manner, sufficient space is made available between two adjacent body elements 10' for accommodating gas analysing means 28 which are received by mounting bore 23.

A desired functionality of the modular gas analyser 2 is attained by receiving parameters relating to the desired functionality wherein said parameters may relate in particular to which components of the gas is to be detected, and based the received parameters proceed to select the appropriate gas analysing means 28 for detecting the said gas G components. The selected gas analysing means 28 is provided with a respecting body element 10' and the body elements 10, 10' arranged in mating sequence, as has been explained herein. The modular gas analyser 2 comprising the gas analyser 1 thus accomplishes that it is customizable to specific applications.

In an exemplary implementation form, the modular gas analyser 2 has a first further conditioning body element 3 comprising a flow regulating valve, such as a magnetic three-way valve, capable of switching a gas flow G to be analysed ON/OFF and/or switching between calibration gas. A second further conditioning body element 3 comprises a flow meter and/or regulating arrangement M. The function of the end piece 4 may be integrated in the second further conditioning body element 3. A third body element 10' comprises gas analysing means 28 which may be in the form of a methane detector, a fourth body element 10' comprises gas analysing means 28 which may be in the form of a carbon dioxide detector, a fifth body element 10' comprises gas analysing means 28 which may be in the form of an oxygen detector and a sixth body element 10' comprises gas analysing means 28 which may be in the form of a hydrogen sulfide detector. The third to sixth body elements 10' may be arranged with body elements 10 interposed between them. The body element 10' comprising the hydrogen sulfide detector is selectively bypassed, i.e. the gas G may be selectively passed through this body element by means of a valve.

According to one aspect, the disclosure also relates to a method for analysing gas. The method comprising the following steps:
- providing one or more body elements 10,
- 10';
- providing each body element 10, 10' with a sample bore 7 extending from a respective proximal surface 11 to a distal surface 12 of said body element 10, 10';
- providing gas conditioning means 25;
- configuring said gas conditioning means to connect with said sample bore 7;
- providing at least one sample bore 7 with an orifice 21 disposed between said proximal surface 11 and said distal surface 12;
- providing gas analysing means 28 in communication with said orifice 21.

Wherein the method may further comprises one or more of the following steps:
- providing each body element 10, 10' with a plurality of independently formed conditioning bores 15;
- interconnecting said conditioning bores 15 to form one or more conditioning channels 19;
- providing gas analysing means 28 in communication with said conditioning channel 19;
- heating said conditioning channel 19 by means of heating means 26;
- providing a gas G to be analysed,
- conditioning the gas to be analysed in respect of pressure and/or volume flow or mass-flow;
- providing the gas G to be analysed to the conditioning channel 19;
- conditioning the gas G in terms of temperature to attain and/or maintain the gas to be analysed at a temperature above a corresponding dew-point temperature of the gas to be analysed;
- analysing the gas to be analysed by means of the gas analysing means 28.
- controlling and/or monitoring one or more of the preceding steps by means of a control system 31.
The step of heating the conditioning channel 19 comprises heating the body element 10, 10'.

Finally it should be mentioned that the invention is by no means limited to the embodiments described above, and many modifications are feasible within the scope of the invention set forth in the appended claims. For instance the gas analyser 1, 2 is suitable for analysing various other gases by means of other gas analysing means than have been mentioned. Further, the body elements 10, 10' may, within the scope of the disclosure, comprise various shapes being assembled in manners which differ from the exemplary embodiments.

## Claims

1. A gas analyser comprising one or more body elements (10, 10'), wherein each body element (10, 10') comprises: a sample bore (7) extending from a respective proximal surface (11) to a distal surface (12) of said body element (10, 10'); and gas conditioning means (25) configured to connect with said sample bore (7); wherein at least one sample bore (7) is provided with an orifice (21) disposed between said proximal surface (11) and said distal surface (12); and wherein gas analysing means (28) is arranged in communication with said orifice (21).

2. The gas analyser according to claim 1, wherein said gas conditioning means (25) are configured to connect with said sample bore (7) upstream of the same.

3. The gas analyser according to claim 1 or 2, wherein said orifice (21) opens to a mounting bore (23) configured to receive and/or hold said gas analysing means (28).

4. The gas analyser according to any one of claims 1 to 3, wherein a gas permeable disk (32) is disposed in said mounting bore (23).

5. The gas analyser according to any one of claims 1 - 4, wherein each body element (10, 10') constitutes one entity.

6. The gas analyser according to any one of claims 1 - 5, wherein said body element (10, 10') is manufactured from a solid casted body.

7. The gas analyser according to any one of claims 1 - 6, wherein said gas conditioning means (25) is configured to condition a gas (G) to be analysed in terms of temperature to attain and/or maintain a temperature above a corresponding dew point temperature of said gas (G) to be analysed.

8. The gas analyser according to any one of claims 1 - 7, wherein said gas conditioning means (25) comprise a plurality of conditioning bores (15), said conditioning bores (15) being configured to be fluidly interconnected.

9. The gas analyser according to claim 8, wherein said conditioning bores (15) are configured to be fluidly interconnected in serial fluid communication to form a continuous conditioning channel (19).

10. The gas analyser according to any one of claims 8 or 9, wherein each conditioning bore (15) extends from said proximal surface (11) to said distal surface (12) of said body element (10, 10').

11. The gas analyser according to any one of claims 1 - 10, wherein respective inner walls (29) of said sample bore (7) and said gas conditioning means (25) are heated by heating means (26) configured to heat said body element (10, 10') during operation.

12. The gas analyser according to any one of claims 8 - 11, wherein said conditioning bores (15) and/or said sample bore (7) extend side by side and in parallel.

13. The gas analyser according to any one of claims 1 - 12, wherein said gas analysing means (28) comprise one or more of a carbon dioxide detector, an oxygen detector, a methane detector, a hydrogen sulfide detector.

14. The gas analyser according to any one of claims 8 - 13, wherein the number of conditioning bores (15) in each respective body element (10, 10') is between four to twelve, preferably six to ten, and most preferred eight.

15. A modular gas analysing assembly, comprising a gas analyser (1) as claimed in any one of the proceeding claims, wherein a desired/required functionality of said modular gas analysing assembly (2) is attained by arranging said one or more body elements (10, 10') in mating sequence to connect respective sample bores (7) and to connect respective conditioning bores (15).
